# EUROPEAN PATENT SPECIFICATION

(11) **EP 2 591 757 B1**
(45) Date of publication and mention of the grant of the patent: **12.08.2015**
(21) Application number: 12191091.3
(22) Date of filing: 02.11.2012
(51) Int. Cl.: A61F 5/37

(54) **Shoulder brace for immobilization of the acromio-clavicular joint**
Schulterbandage zur Immobilisierung des Akromioklavikulargelenks
Support d'épaule pour l'immobilisation de l'articulation acromio-claviculaire

(30) Priority: 08.11.2011 IT MI20112022
(43) Date of publication of application: 15.05.2013
(73) Proprietor: Orthoservice AG, 6830 Chiasso (CH)
(72) Inventor: Bernareggi, Aldo, 20842 Besana in Brianza (MB) (IT); Turconi, Francesco, 20900 Monza (MB) (IT); Ravese, Mauro, 20856 Correzzana (MB) (IT)
(74) Representative: Branca, Emanuela

(56) References cited:
- EP-A1- 0 425 938
- BE-A2- 897 621
- US-A- 4 198 964
- US-A1- 2004 215 119
- US-A1- 2005 020 950

## Description

The present invention relates to an improved brace for immobilization of the acromioclavicular joint in the shoulder.

In particular, the brace is adapted to stabilize the shoulder and the arm in cases of luxuation and subluxuation of the acromioclavicular joint.

Traumas of the acromioclavicular joint, due to falls, for example, can determine the separation of the acromion from the clavicle causing the spraining, partial tearing or rupture of the acromioclavicular ligament and, possibly, of the coracoclavicular ligaments also.

The brace acts by lowering the clavicle to realign it with the acromion and lifting the humerus to promote realignment in the joint to be treated so as to allow the soft tissues to heal and restore correct joint functionality in the course of around 2/3 weeks.

The brace must, for such purposes, exert a pressure on the upper part of the shoulder and support, in proximity of the elbow, the forearm held in a folded position adherent to the torso to avoid the risk of extra rotating the shoulder. Additionally, the shoulder must be neither abducted nor adducted.

Braces of this type, comprising a sleeve for the forearm and a shoulder pressure pad connected to the sleeve by means of a front strap and a rear strap aligned to the shoulder pressure pad, are known in the prior art.

It is essential that the shoulder pressure pad and the relevant straps aligned thereto of all braces for the immobilization of the acromioclavicular joint, which support the sleeve in proximity of the immobilized elbow, act on the shoulder to be treated.

The braces of the prior art also comprise a traversal band, arranged almost horizontally, which connects the front side and the rear side of the shoulder pressure pad together passing under the armpit of the arm opposite, the purpose of which is to keep the brace in the correct position when worn and, in particular, to prevent the shoulder pressure pad from slipping down off the shoulder.

The end of the sleeve facing the hand is generally hung to the traversal band to obtain a further support point for the forearm.

One of the main technical drawbacks of acromioclavicular braces, as yet inadequately resolved, relates to the distribution of loads due to the weight of the forearm. Indeed, it is on the one hand necessary to optimize the action on the joint but it is also desirable, on the other hand, to take into patient comfort into account.

A further drawback of the braces of the prior art relates to the difficulty in keeping the forearm in a position adherent to the body while avoiding rotations.

The braces of the prior art also require the intervention of auxiliary staff to put on the brace, both because the operation is laborious for patients with reduced mobility and because it generally requires specific adjustments to be made every time that the brace is put on.

Lastly, one aspect that is normally overlooked by the braces of the prior art is the maintenance of the correct position and stress on the shoulder to be treated when the patient is in a horizontal position, position in which the action of gravity of the arm itself is not exploited to lower the clavicle.

The document BE 897 621 A shows a brace for the acromioclavicular joint in the shoulder comprising a horizontal band placed at the lower edge of the torso, to which a sleeve for the elbow is attached, and to which a cuff is hung, which supports the patient's wrist against the action of the force of gravity. The brace also comprises a substantially horizontal strap passing below the patient's armpit connected to a strap that is perpendicular thereto. Said substantially vertical strap is equipped with a shoulder pressure pad and is affixed in its lower part to the elbow sleeve.

Due to its structure, said brace is nevertheless able to effectively prevent the shoulder pressure pad slipping from the patient's shoulder. In addition, the wrist being hung to the vertical strap, does not allow the arm to be kept still, and thus the joint to be immobilized, for example the supine position where the force of gravity cannot be exploited.

The aim of the present invention is that of producing an improved brace for the immobilization of the acromioclavicular joint in the shoulder that overcomes the aforementioned drawbacks.

Another aim of the present invention is that of producing an improved brace for the immobilization of the acromioclavicular joint in the shoulder that is easy to put on, even by the same patient, and is simple to adjust by persons skilled in the art.

A further aim is to produce an improved brace for the immobilization of the acromioclavicular joint in the shoulder that is effective even in a supine position.

Another aim of the present invention is that of producing an improved brace for the immobilization of the acromioclavicular joint in the shoulder that is particularly simple and functional, at contained costs.

These aims according to the present invention are achieved by producing an improved brace for the immobilization of the acromioclavicular joint in the shoulder as set out in claim 1.

Further characteristics are envisaged in the dependent claims.

The characteristics and the advantages of an improved brace for the immobilization of the acromioclavicular joint in the shoulder according to the present invention will become clearer from the below description, provided by way of a non-limiting example, of the accompanying schematic drawings, wherein:

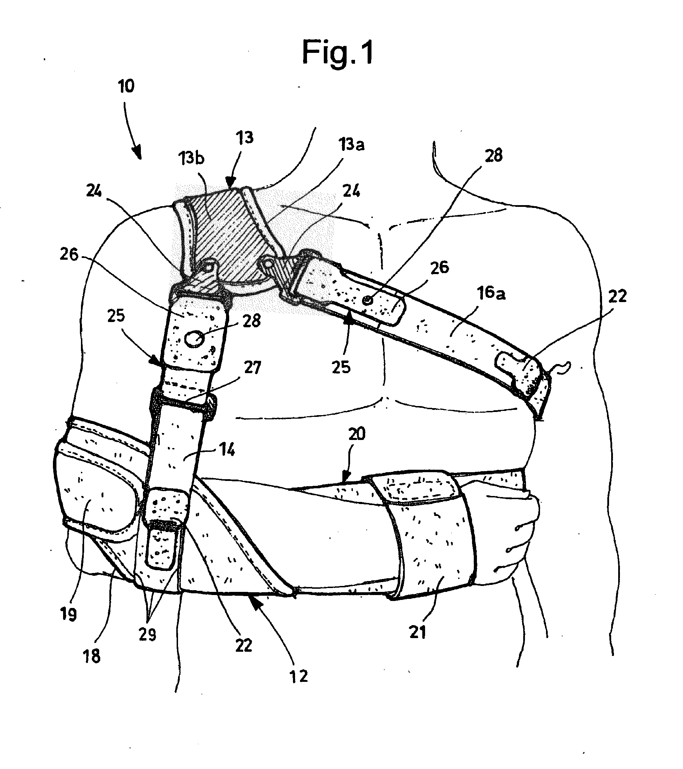

figure 1 shows a front view of an improved brace for immobilizing the acromioclavicular joint in the shoulder, according to the invention, worn by a patient;
figure 2 shows the brace of figure 1, wherein the straps are shown open for brace removal;
figure 3 is a side view of the brace of figure 1, wherein the straps are partially open;

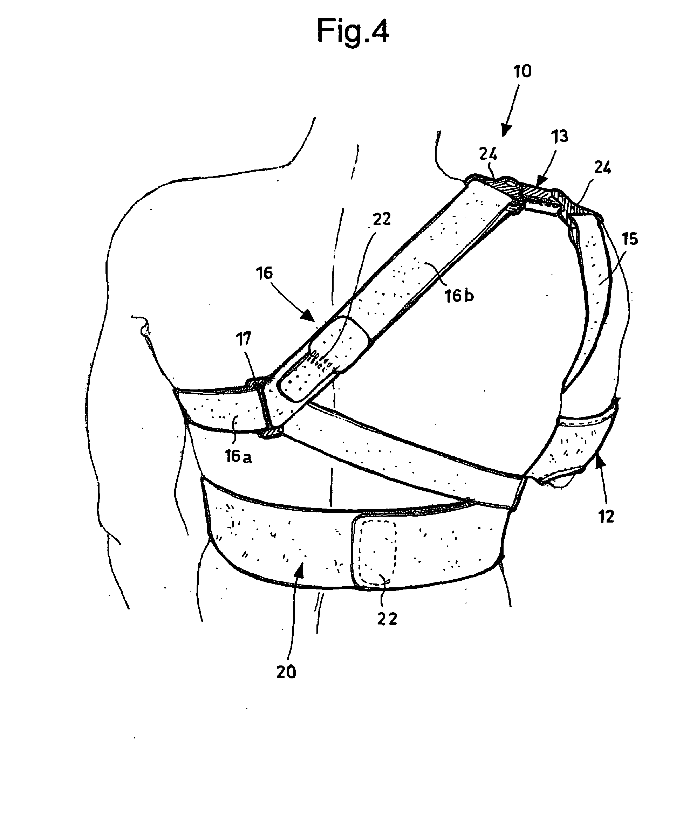

figure 4 shows a rear view of the brace for immobilizing the acromioclavicular joint in the shoulder, according to the invention, worn by a patient;

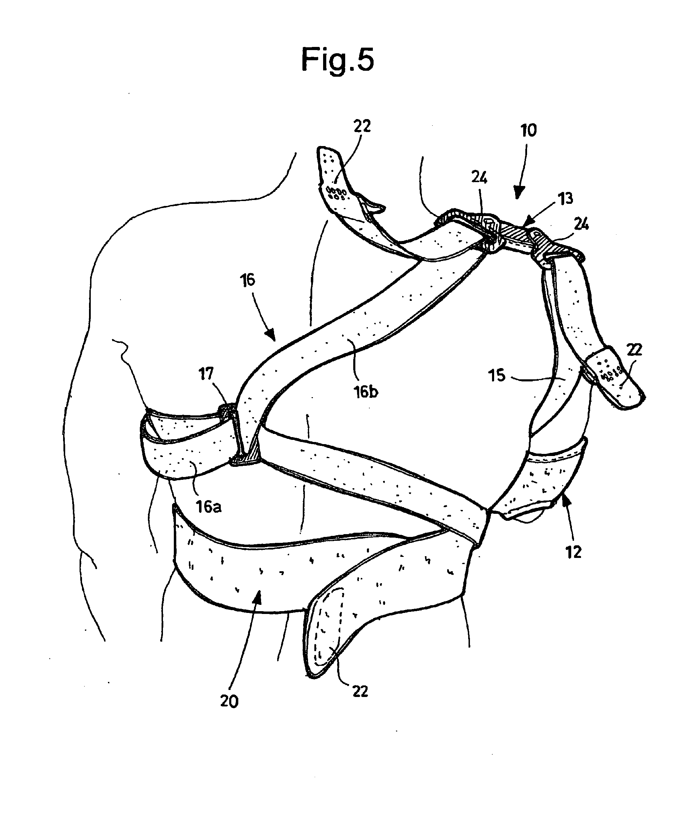

figure 5 shows a rear view of the brace for immobilizing the acromioclavicular joint in the shoulder, according to the invention, worn by a patient, wherein the straps are shown partially open for brace removal;

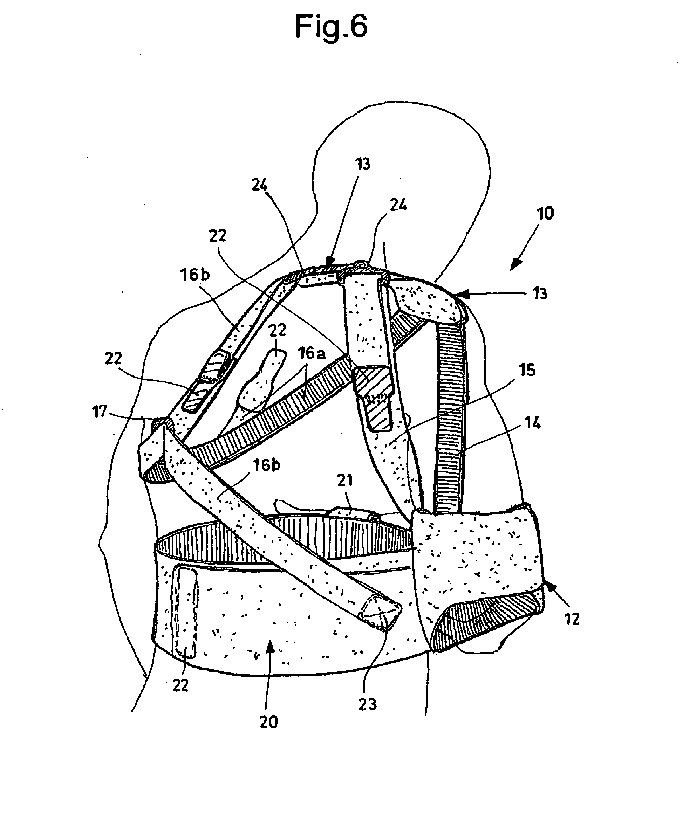

figure 6 shows a rear view of the brace according to the invention worn by a transparent mannequin to show the inner side of the brace;
figure 7 shows a detail of the fastening of the elbow sleeve and of the rear tensioning strap shown from the inner side of the brace according to the invention;

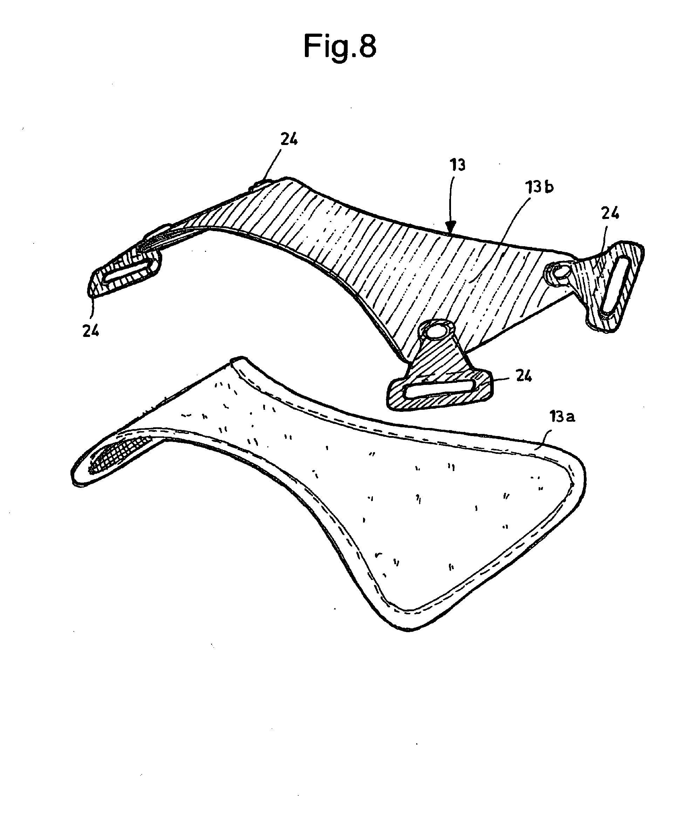

figure 8 shows an exploded detail of the shoulder pressure pad of the brace according to the invention;
figure 9 shows a further variant embodiment of the tensioning joint in the brace according to the invention.

With reference to the drawings, these show an improved brace for the immobilization of the acromioclavicular joint, overall indicated by 10, comprising a sleeve 12 and a shoulder pressure pad 13, adapted to respectively intervene on elbow and shoulder of the same side of the body, whether right (in the example shown) or left. The brace 10 is not ambidextrous, that is to say, a brace for the immobilization of the acromioclavicular joint in the left shoulder will be constructed in specular manner to what has been shown.

The brace 10 also comprises a front tensioning strap 14 and a rear tensioning strap 15, vertically arranged between the shoulder pressure pad 13 and a front and rear side of the sleeve 12 respectively.

The brace 10 further comprises diagonal strap retention members 16, connectable to the shoulder pressure pad 13 at the front and rear and positionable around the torso and under the arm opposite the one being treated.

The brace 10 according to the invention further comprises a circumferential waistband 20, that can be circumferentially tightened in an adjustable manner around the patient's waist in an abdominal position, onto which the elbow sleeve 12, the front tensioning strap 14 and the rear tensioning strap 15 are firmly connected.

The connections between the sleeve 12 and the waistband 20 and/or the connection between the front tensioning strap 14 the rear tensioning strap 15 and the waistband 20 can take place directly or indirectly.

Preferably, the sleeve 12 and the front tensioning strap 14 and the rear tensioning strap 15 are each sewn directly onto the waistband 20.

In addition, according to a further preferred embodiment shown in the figures, the front tensioning strap 14 and the rear tensioning strap 15 are part of a single strap that is also firmly constrained, as well as to the waistband 20, to the lower side of sleeve 12, an in particular sewn to it along the dotted lines 29.

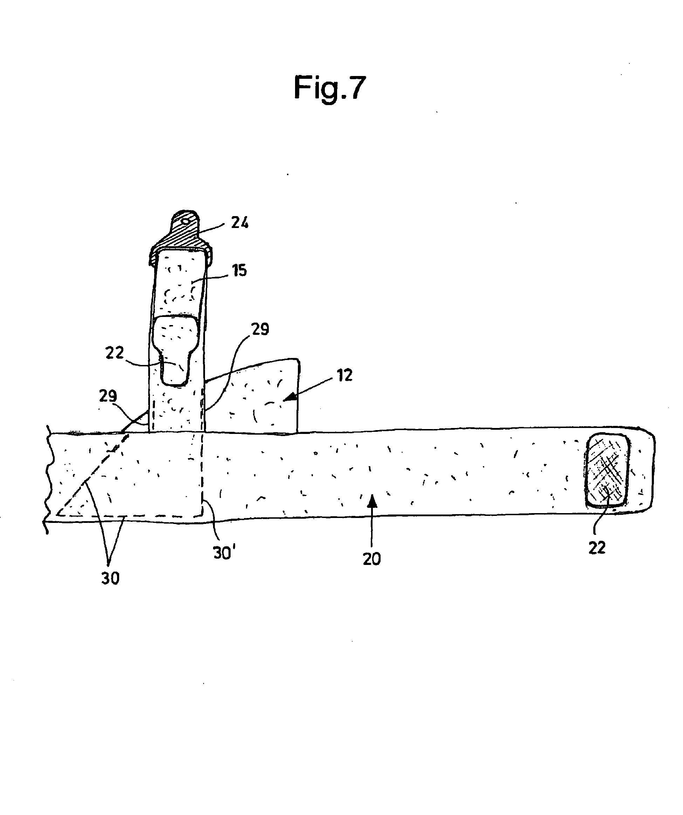

In this case, the sleeve 12 is also in turn firmly constrained by means of stitching to the waistband 20, schematically shown in figure 7 with the dotted lines 30.

The particular creation of at least one stitching 30' that constrains all the elements together, arranged vertically along the joint to be treated, gives the brace greater stability.

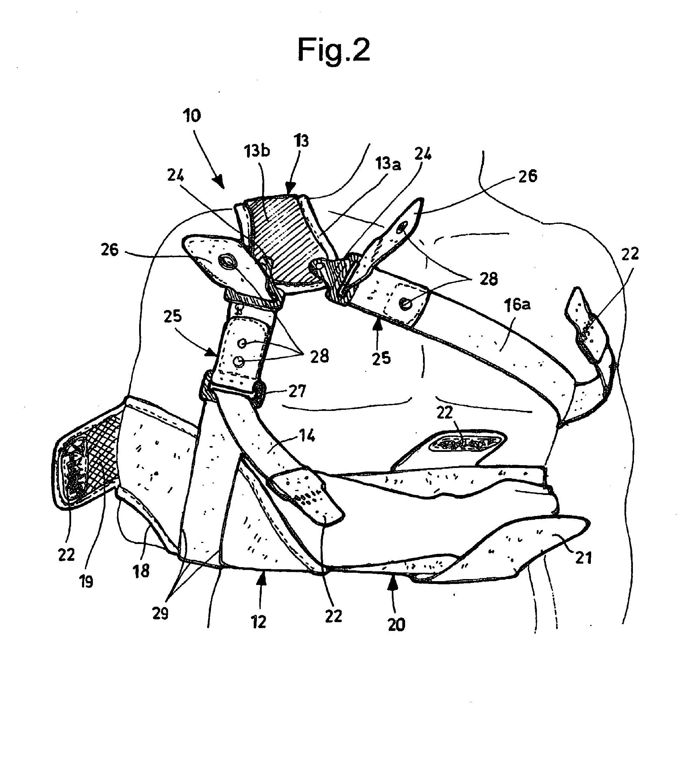

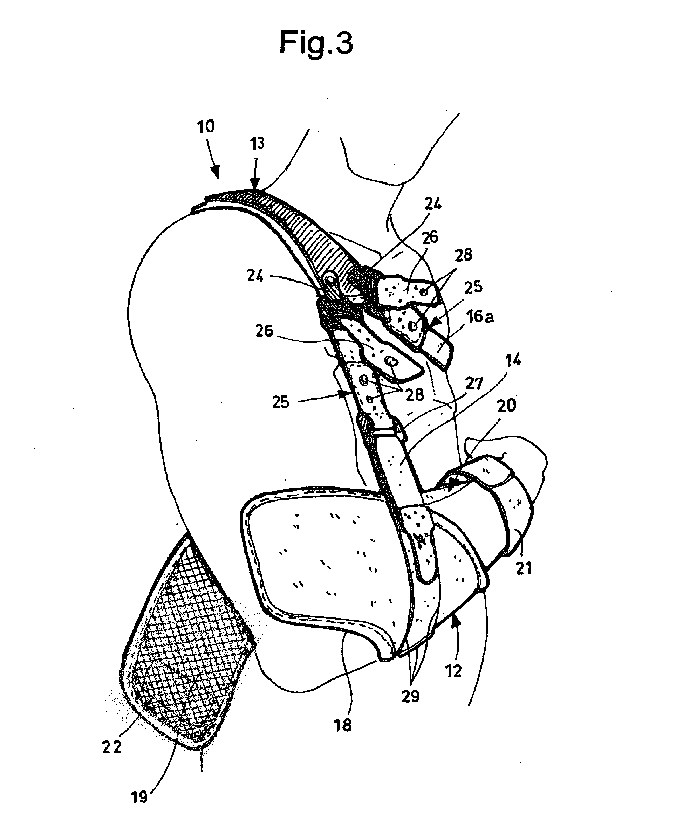

As shown in figures 2 and 3, the elbow sleeve 12 is provided with an opening 18 at the end facing towards the elbow bearing adjustable closure means 19.

The waistband 20 also bears retention means 21 for the free end of the treated arm, which constrain the hand or the wrist into keeping as still a position as possible next to the waistband 20 and thus to the torso even in a supine position, to prevent flexing of the forearm.

The retention means 21 consist, according to one preferred embodiment, of an adjustable cuff, both in terms of circumferential dimensions and positioning along the waistband 20 to allow the best possible adjustment in respect of each patient's anatomy.

The waistband 20 can, for example, be made of fabric that can be coupled with gripping means 22 for a Velcro^{®} type coupling.

Making the waistband 20 with fabric also allows the stitching 29 to be made for the firm constraint with the tensioning straps 14, 15 and with the sleeve 12.

In order to obtain the circumferential closure of the waistband 20 according to the optimal adjustment for the patient, the waistband 20 is equipped at one end with gripping means 22 that can engage with the waistband 20 at any point of the circumference.

Similarly, the adjustable cuff 21 for retaining the free end of the arm is also applied to the waistband 20 in the positions selected from time to time through the gripping means 22. Preferably, the cuff 21 itself is made of fabric that can be coupled with the gripping means 22 for a Velcro^{®} type coupling. This allows the circumferential dimension to be adjusted.

According to a preferred embodiment of the brace 10 according to the invention, the diagonal strap retention members 16, are also firmly constrained to a fixed point 23 of the waistband 20, through stitching for example. This precaution improves their function of preventing the shoulder pressure pad from slipping, limits the possibility of rotation of the arm and increases comfort for the patient as it prevents the diagonal strap retention members 16 from positioning themselves too high on the torso and, in particular, directly beneath the armpit of the arm opposite the one being treated.

According to one preferred embodiment of the present invention, the diagonal strap retention members 16 comprise a front diagonal strap 16a and a rear diagonal strap 16b that converge in a rear transmission eyelet 17.

The front diagonal strap 16a has a first end connectable to the shoulder pressure pad 13 and a second end engageable with a variable length in the rear transmission eyelet 17.

The rear diagonal strap 16b has a first end firmly constrained to the waistband 20 at the fixed point 23, is transmitted into the rear transmission eyelet 17 and has a second end connectable with adjustable length to the shoulder pressure pad 13 in a rear position.

In order to improve the adaptability of the brace 10 to the different patient features, all the straps, that is to say, in the example show, the front tensioning strap 14 and the rear tensioning strap 15, as well as the diagonal strap retention members 16, are all advantageously hinged in their respective point of connection with the shoulder pressure pad 13, preferably through a hinged transmission eyelet 24.

In order to give greater mechanical resistance, the shoulder pressure pad 13 is, according to one preferred embodiment, made in two layers that are connectable together, that is to say, it comprises a padding 13a in its lower part and a reinforcing plate 13b, in plastic for example, in its upper part.

Advantageously, at least the front tensioning strap 14 is hinged to the shoulder pressure pad 13 by means of interposition of a tensioning joint 25. According to the embodiment shown, the front diagonal strap 16a is also hinged to the shoulder pressure pad 13 by interposition of a tensioning joint 25.

The tensioning joint 25 comprises a tensioner 26, the first end of which is rendered integral to the respective front tensioning strap 14 or to the front diagonal strap 16a, for example respectively through a front transmission eyelet 27 or directly sewn thereto.

The opposite end of the tensioner 26 is, on the other hand, transmitted into the transmission eyelet 24 hinged to the shoulder pressure pad 13.

The tensioner 26 is also respectively provided, in a central portion and at the end transmitted into the eyelet 24, with adjustable quick release means 28 for the closure in one or more discrete positions. The tensioner 26 can be held firmly in the eyelet 24 hinged to the shoulder pressure pad 13 to prevent a full and pointless disassembly of the brace 10, or can be removable therefrom.

The adjustable quick release means 28 allow modification of the length of the front tensioning strap 14 or of the diagonal front strap 16a, and thus respectively of the distance between the shoulder pressure pad 13 and the sleeve 12 or the width of the diagonal strap members 16, at least between a first open position of maximum distance, adapted to allow the brace 10 to be put on, and a second closed position of minimum distance, during use.

This allows the patient to put on the brace 10 without modifying the strap length adjustments carried out by personnel skilled in the art.

According to a preferred embodiment of the brace 10 according to the invention, the tensioner 26 of the tensioning joint 25 of the front tensioning strap 14 is firmly held in the eyelet 24 and has two discrete adjustment positions, obtained through automatic buttons for example. The tensioner 26 of the tensioning joint 25 of the diagonal front strap 16a is, on the other hand, preferably releasable from the eyelet 24.

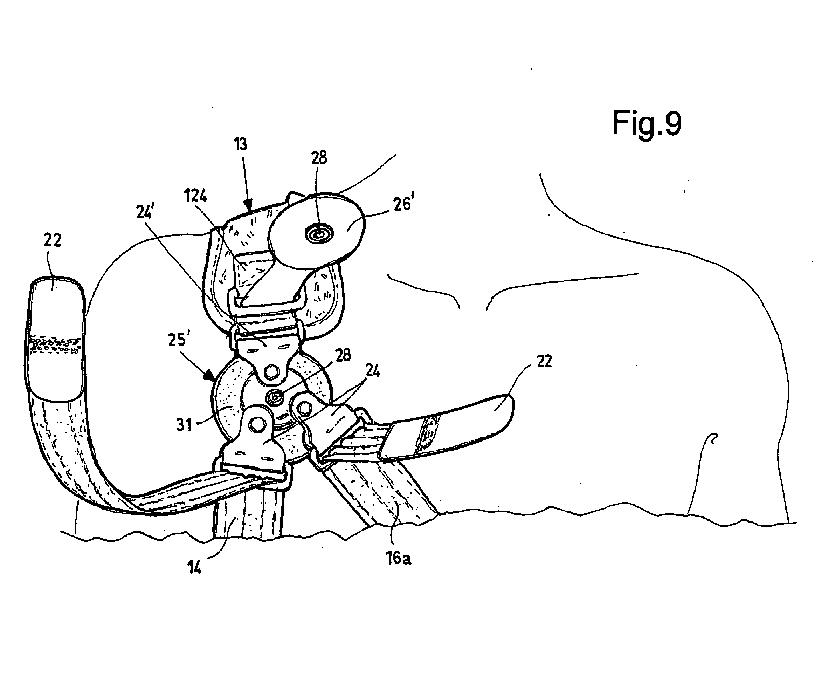

Figure 9 shows a further variant embodiment of the tensioning joint 25' in the brace 10 according to the invention, able to tension both front straps 14 and 16a directly. According to this variant embodiment, the end of the tensioner 26' equipped with the adjustable quick release means 28 is transmitted into an eyelet 124 that is integral to the shoulder pressure pad 13. The opposite end of the tensioner 26' is hinged to a central articulation 31 provided with the adjustable quick release means 28. The front tensioning strap 14 and the diagonal front strap 16a are both also hinged to the central articulation 31.

In the improved brace for the immobilization of the acromioclavicular joint in the shoulder according to the invention, the front tensioning strap 14 and the rear tensioning strap 15 each have one end with adjustable length.

For adjustment of the length, each of the straps has gripping means 22 at the end transmitted into the respective eyelet, that is to say, the front transmission eyelet 27 or rear transmission eyelet 17 or the eyelet 24 hinged to the shoulder pressure pad, wherein the gripping means 22 can be coupled with relevant portions of fabric for a Velcro^{®} type coupling. Preferably, all the straps are wholly made of fabric that can be coupled with the gripping means 22 for a Velcro^{®} type coupling to allow a continuous adjustment of the length, that is to say, in any position, instead of in discrete positions.

According to the preferred embodiment shown in the figures, the end with adjustable length of the front tensioning strap 14 is transmitted into the front eyelet 27 connecting to the tensioning joint 25; the diagonal front strap 16a has the end with adjustable length transmitted into the rear transmission eyelet 17 positioned on the patient's back; the rear tensioning strap 15 and diagonal rear strap 16b each have the end with adjustable length that is transmitted into the eyelets 24 hinged onto the rear side of the shoulder pressure pad 13.

The improved brace for immobilization of the acromioclavicular joint in the shoulder, object of the present invention, has the advantage of exercising the same action on the joint to be treated when the patient is in an upright position and when in a supine position.

The presence of the diagonal straps has the advantage of preventing the rotation of the brace, giving comfort and preventing the shoulder pressure pad from slipping.

The adjustable quick release means that allow modification of the length of the front straps allow the brace to be opened for its removal without modifying the remaining adjustments.

They also advantageously act as a winch thus reducing the required fastening force and assisting patients to put on the brace on their own.

The improved brace for immobilization of the acromioclavicular joint in the shoulder thus conceived is susceptible of numerous modifications and variants, which all fall under the invention. In practice, any materials, as well as any dimensions, can be used depending on the technical requirements.

## Claims

1. Brace for immobilization of the acromioclavicular joint in the shoulder comprising a sleeve (12) for the elbow and a shoulder pressure pad (13), adapted to respectively intervene on the elbow and shoulder on the same side of the body, whether right or left, a front tensioning strap (14) and a rear tensioning strap (15), arranged vertically between said shoulder pressure pad (13) and a front side and rear side of said sleeve (12) respectively, strap retention members (16), positionable around the torso and under the arm opposite to the one being treated, as well as a waistband (20), **characterized in that** said sleeve (12) for the elbow and said front tensioning strap (14) and rear tensioning strap (15) are firmly constrained, in a direct or indirect manner, to said waistband (20), wherein said strap retention members (16) are connected to said shoulder pressure pad (13) both at the front and at the back and positioned diagonally, as well as **in that** said waistband (20) can be tightened circumferentially in an adjustable manner in an abdominal position around the waist, said waistband (20) also presenting retention means (21) for firmly positioning the free end of the arm being treated next to said waistband (20).

2. Brace according to claim 1, **characterized in that** said diagonal strap retention members (16) are hinged to said shoulder pressure pad (13).

3. Brace according to claims 1 or 2, **characterized in that** said diagonal strap retention members (16) are also firmly constrained at a fixed point (23) of said waistband (20).

4. Brace according to claim 3, **characterized in that** said diagonal strap retention members (16) comprise a front diagonal strap (16a) and a rear diagonal strap (16b) that converge in a rear transmission eyelet (17).

5. Brace according to claim 4, **characterized in that** said front diagonal strap (16a) has a first end connectable to said shoulder pressure pad (13) and a second end with adjustable length engageable in said rear transmission eyelet (17) and that said rear diagonal strap (16b) has a first end firmly constrained to said waistband (20), is transmitted to said rear eyelet (17) and has a second end with adjustable length connectable to said shoulder pressure pad (13).

6. Brace according to claim 5, **characterized in that** said ends connectable to said shoulder pressure pad (13) of said diagonal front strap (16a) and rear strap (16b) are hinged.

7. Brace according to any one of the preceding claims, **characterized in that** said front tensioning strap (14) and rear tensioning strap (15) each have one end with adjustable length hinged to said shoulder pressure pad (13).

8. Brace according to claim 7, **characterized in that** at least said front tensioning strap (14) is hinged to said shoulder pressure pad (13) by means of interposition of a tensioning joint (25, 25'), wherein said tensioning joint (25, 25'), comprises adjustable quick-release means (28), which allow modification of the length of the strap at least between a first open position adapted to allow the brace to be put on, and a second closed position during use.

9. Brace according to claim 8, **characterized in that** said diagonal strap retention members (16) are also hinged at the front to said shoulder pressure pad (13) by means of interposition of said tensioning joint (25, 25').

10. Brace according to claims 8 or 9, **characterised in that** said tensioning joint (25) comprises a tensioner (26), the first end of which is connectable to said front tensioning strap (14) or to said diagonal strap retention members (16) and wherein said tensioner (26) bears in a central portion, and on the opposite end, said adjustable quick-release means (28), wherein said opposite end is transmitted to an eyelet (24) that is hinged to said shoulder pressure pad (13).

11. Brace according to any one of the preceding claims, **characterized in that** said front tensioning strap (14) and said rear tensioning strap (15) are part of a single strap that is firmly constrained to the lower side of said sleeve (12) as well as to said waistband (20).

12. Brace according to any one of the preceding claims **characterized in that** said shoulder pressure pad (13) comprises two layers connectable to each other, consisting in their lower part of a padding (13a) and in their upper part of a reinforcing plate (13b).

13. Brace according to any one of the preceding claims, **characterized in that** said retention means (21) for the free end of the arm consist of an adjustable cuff which is positionable at any point along said waistband (20) to retain the arm in the proximity of the wrist.

14. Brace according to claim 13, **characterized in that** said retention means (21) are positioned to form a ring around said waistband (20) to envelop the wrist, tightening it in contact with said waistband (20).

## Patentansprüche

1. Bandage zur Immobilisierung des Akromioklavikulargelenks in der Schulter, umfassend eine Manschette (12) für den Ellenbogen und eine Schulterdruckpelotte (13), die geeignet sind, am Ellenbogen beziehungsweise an der Schulter auf derselben Seite des Körpers, gleichermaßen rechts oder links, anzugreifen, einen vorderen Spanngurt (14) und einen hinteren Spanngurt (15), die senkrecht zwischen der Schulterdruckpelotte (13) und einer Vorderseite beziehungsweise Rückseite der Manschette (12) angeordnet sind, Gurtrückhalteelemente (16), die um den Rumpf und unter dem Arm, der dem behandelten Arm entgegengesetzt ist, angeordnet werden können, sowie ein Taillenband (20), **dadurch gekennzeichnet, dass** die Manschette (12) für den Ellenbogen und der vordere Spanngurt (14) und der hintere Spanngurt (15) direkt oder indirekt fest an dem Taillenband (20) befestigt sind, wobei die Gurtrückhalteelemente (16) sowohl auf der Vorderseite als auch auf der Rückseite mit der Schulterdruckpelotte (13) verbunden und diagonal angeordnet sind, und dadurch, dass das Taillenband (20) zirkumferenziell in einstellbarer Weise in einer abdominalen Lage um die Taille gespannt werden kann, wobei das Taillenband (20) außerdem Haltemittel (21) zum festen Anordnen des freien Endes des behandelten Arms nahe dem Taillenband (20) aufweist.

2. Bandage nach Anspruch 1, **dadurch gekennzeichnet, dass** die diagonalen Gurtrückhalteelemente (16) an die Schulterdruckpelotte (13) angelenkt sind.

3. Bandage nach Anspruch 1 oder 2, **dadurch gekennzeichnet, dass** die diagonalen Gurtrückhalteelemente (16) außerdem fest an einem festen Punkt (23) des Taillenbands (20) befestigt sind.

4. Bandage nach Anspruch 3, **dadurch gekennzeichnet, dass** die diagonalen Gurtrückhalteelemente (16) einen vorderen diagonalen Gurt (16a) und einen hinteren diagonalen Gurt (16b) umfassen, die in einer hinteren Leitöse (17) zusammenlaufen.

5. Bandage nach Anspruch 4, **dadurch gekennzeichnet, dass** der vordere diagonale Gurt (16a) ein erstes Ende, das mit der Schulterdruckpelotte (13) verbunden werden kann, und ein zweites Ende mit einstellbarer Länge aufweist, das mit der hinteren Leitöse (17) in Eingriff gebracht werden kann, und dass der hintere diagonale Gurt (16b) ein fest mit dem Taillenband (20) verbundenes erstes Ende aufweist, das zur hinteren Öse (17) geleitet wird, und ein zweites Ende mit einstellbarer Länge aufweist, das mit der Schulterdruckpelotte (13) verbunden werden kann.

6. Bandage nach Anspruch 5, **dadurch gekennzeichnet, dass** die mit der Schulterdruckpelotte (13) verbindbaren Enden des diagonalen vorderen Gurts (16a) und hinteren Gurts (16b) angelenkt sind.

7. Bandage nach einem der vorhergehenden Ansprüche, **dadurch gekennzeichnet, dass** der vordere Spanngurt (14) und der hintere Spanngurt (15) jeweils ein Ende mit einstellbarer Länge aufweisen, das an die Schulterdruckpelotte (13) angelenkt ist.

8. Bandage nach Anspruch 7, **dadurch gekennzeichnet, dass** zumindest der vordere Spanngurt (14) an die Schulterdruckpelotte (13) durch Einfügen eines Spanngelenks (25, 25') angelenkt ist, wobei dieses Spanngelenk (25, 25') eine einstellbare Schnelllöseeinrichtung (28) umfasst, die das Ändern der Länge des Gurts zumindest zwischen einer ersten offenen Stellung, die geeignet ist, das Anlegen der Bandage zu ermöglichen, und einer zweiten geschlossenen Stellung während des Gebrauchs ermöglicht.

9. Bandage nach Anspruch 8, **dadurch gekennzeichnet, dass** die diagonalen Gurtrückhalteelemente (16) auch durch Einfügen des Spanngelenks (25, 25') an die Vorderseite der Schulterdruckpelotte (13) angelenkt sind.

10. Bandage nach Anspruch 8 oder 9, **dadurch gekennzeichnet, dass** das Spanngelenk (25) einen Spanner (26) umfasst, dessen erstes Ende mit dem vorderen Spanngurt (14) oder mit den diagonalen Gurtrückhalteelemente (16) verbunden werden kann, und wobei dieser Spanner (26) in einem mittleren Abschnitt und auf dem entgegengesetzten Ende die einstellbare Schnelllöseeinrichtung (28) trägt, wobei dieses entgegengesetzte Ende zu einer Öse (24) geleitet wird, die an die Schulterdruckpelotte (13) angelenkt ist.

11. Bandage nach einem der vorhergehenden Ansprüche, **dadurch gekennzeichnet, dass** der vordere Spanngurt (14) und der hintere Spanngurt (15) Teil eines einzigen Gurts sind, der fest an der unteren Seite der Manschette (12) und am Taillenband (20) befestigt ist.

12. Bandage nach einem der vorhergehenden Ansprüche, **dadurch gekennzeichnet, dass** die Schulterdruckpelotte (13) zwei miteinander verbindbare Lagen umfasst, die in ihrem unteren Teil aus einer Polsterung (13a) und in ihrem oberen Teil aus einer Verstärkungsplatte (13b) bestehen.

13. Bandage nach einem der vorhergehenden Ansprüche, **dadurch gekennzeichnet, dass** die Haltemittel (21) für das freie Ende des Arms aus einer einstellbaren Manschette bestehen, die an jedem Punkt längs des Taillenbands (20) angeordnet werden kann, um den Arm in der Nähe des Handgelenks festzuhalten.

14. Bandage nach Anspruch 13, **dadurch gekennzeichnet, dass** die Haltemittel (21) derart angeordnet sind, dass sie einen Ring um das Taillenband (20) bilden, um das Handgelenk zu umfassen, wobei sie es in Kontakt mit dem Taillenband (20) festziehen.

## Revendications

1. Support pour l'immobilisation de l'articulation acromio-claviculaire dans l'épaule, comprenant un manchon (12) pour le coude et un coussinet de pression d'épaule (13), adapté pour intervenir respectivement sur le coude et l'épaule sur le même côté du corps, droit ou gauche, une sangle de tension avant (14) et une sangle de tension arrière (15), disposées verticalement entre ledit coussinet de pression d'épaule (13) et respectivement un côté avant et un côté arrière dudit manchon (12), des éléments de retenue de sangle (16), positionnables autour du torse et sous le bras opposé à celui qui est traité, ainsi qu'une ceinture (20), **caractérisé en ce que** ledit manchon (12) pour le coude et ladite sangle de tension avant (14) et ladite sangle de tension arrière (15) sont fixées solidement, d'une manière directe ou indirecte, à ladite ceinture (20), dans lequel lesdits éléments de retenue de sangle (16) sont connectés audit coussinet de pression d'épaule (13) à la fois à l'avant et à l'arrière et positionnés en diagonale, et **en ce que** ladite ceinture (20) peut être serrée de manière circonférentielle d'une manière réglable dans une position abdominale autour de la taille, ladite ceinture (20) présentant également des moyens de retenue (21) pour positionner fermement l'extrémité libre du bras traité à côté de ladite ceinture (20).

2. Support selon la revendication 1, **caractérisé en ce que** lesdits éléments de retenue de sangle en diagonale (16) sont articulés audit coussinet de pression d'épaule (13).

3. Support selon la revendication 1 ou 2, **caractérisé en ce que** lesdits éléments de retenue de sangle en diagonale (16) sont également fixés solidement en un point de fixation (23) de ladite ceinture (20).

4. Support selon la revendication 3, **caractérisé en ce que** lesdits éléments de retenue de sangle en diagonale (16) comprennent une sangle diagonale avant (16a) et une sangle diagonale arrière (16b) qui convergent dans un oeillet de transmission arrière (17).

5. Support selon la revendication 4, **caractérisé en ce que** ladite sangle diagonale avant (16a) a une première extrémité pouvant être connectée audit coussinet de pression d'épaule (13) et une deuxième extrémité avec longueur réglable pouvant être engagée dans ledit oeillet de transmission arrière (17) et **en ce que** ladite sangle diagonale arrière (16b) a une première extrémité fixée solidement à ladite ceinture (20), et est transmise audit oeillet arrière (17) et a une deuxième extrémité avec longueur réglable pouvant être connectée audit coussinet de pression d'épaule (13).

6. Support selon la revendication 5, **caractérisé en ce que** lesdites extrémités pouvant être connectées audit coussinet de pression d'épaule (13) de ladite sangle diagonale avant (16a) et ladite sangle diagonale arrière (16b) sont articulées.

7. Support selon l'une quelconque des revendications précédentes, **caractérisé en ce que** ladite sangle de tension avant (14) et ladite sangle de tension arrière (15) ont chacune une extrémité avec longueur réglable articulée audit coussinet de pression d'épaule (13).

8. Support selon la revendication 7, **caractérisé en ce qu'**au moins ladite sangle de tension avant (14) est articulée audit coussinet de pression d'épaule (13) par le biais de l'interposition d'un joint de tension (25, 25'), dans lequel ledit joint de tension (25, 25') comprend des moyens de décrochage rapide réglables (28) qui permettent la modification de la longueur de la sangle au moins entre une première position ouverte adaptée pour permettre de mettre en place le support et une deuxième position fermée durant l'utilisation.

9. Support selon la revendication 8, **caractérisé en ce que** lesdits éléments de retenue de sangle en diagonale (16) sont également articulés à l'avant audit coussinet de pression d'épaule (13) par le biais de l'interposition dudit joint de tension (25, 25').

10. Support selon la revendication 8 ou 9, **caractérisé en ce que** ledit joint de tension (25) comprend un tendeur (26), dont la première extrémité peut être connectée à ladite sangle de tension avant (14) ou auxdits éléments de retenue de sangle diagonaux (16) et dans lequel ledit tendeur (26) porte dans une position centrale, et sur l'extrémité opposée, lesdits moyens de décrochage rapide réglables (28), dans lequel ladite extrémité opposée est transmise à un oeillet (24) qui est articulé audit coussinet de pression d'épaule (13).

11. Support selon l'une quelconque des revendications précédentes, **caractérisé en ce que** ladite sangle de tension avant (14) et ladite sangle de tension arrière (15) font partie d'une unique sangle qui est fixée solidement au côté inférieur dudit manchon (12) ainsi qu'à ladite ceinture (20).

12. Support selon l'une quelconque des revendications précédentes, **caractérisé en ce que** ledit coussinet de pression d'épaule (13) comprend deux couches pouvant être connectées entre elles, comprenant dans leur partie inférieure un rembourrage (13a) et dans leur partie supérieure une plaque de renforcement (13b).

13. Support selon l'une quelconque des revendications précédentes, **caractérisé en ce que** lesdits moyens de retenue (21) pour l'extrémité libre du bras comprennent une manchette réglable qui est positionnable en n'importe quel point le long de ladite ceinture (20) pour retenir le bras à proximité du poignet.

14. Support selon la revendication 13, **caractérisé en ce que** lesdits moyens de retenue (21) sont positionnés pour former un anneau autour de ladite ceinture (20) pour envelopper le poignet, en le serrant en contact avec ladite ceinture (20).
